# EUROPEAN PATENT SPECIFICATION

(11) **EP 3 232 257 B1**
(45) Date of publication and mention of the grant of the patent: **09.12.2020**
(21) Application number: 15832783.3
(22) Date of filing: 31.08.2015
(51) Int. Cl.: G02F 1/133, G09F 9/00, G02F 1/1333

(54) **MULTI-SIDED DISPLAY DEVICE**
MEHRSEITIGE ANZEIGEVORRICHTUNG
DISPOSITIF D'AFFICHAGE À CÔTÉS MULTIPLES

(30) Priority: 12.12.2014 CN 201410771575
(43) Date of publication of application: 18.10.2017
(73) Proprietor: BOE Technology Group Co., Ltd., Beijing 100015 (CN); Beijing BOE Optoelectronics Technology Co., Ltd., Beijing 100176 (CN)
(72) Inventor: WANG, Jiaheng, Beijing 100176 (CN); BAI, Feng, Beijing 100176 (CN); YANG, Jiuxia, Beijing 100176 (CN)
(74) Representative: Brötz, Helmut
(86) International application number: PCT/CN2015/088602
(87) International publication number: WO 2016/090974

(56) References cited:
- EP-A1- 1 376 199
- WO-A1-2014/007555
- CN-A- 103 871 329
- CN-A- 104 460 087
- CN-A- 104 460 088
- CN-U- 203 275 845
- CN-U- 204 229 081
- CN-U- 204 241 801
- CN-Y- 2 378 796
- JP-A- 2012 141 341
- US-A1- 2007 285 413
- US-A1- 2009 082 103
- US-A1- 2013 155 717
- US-A1- 2014 152 646
- US-A1- 2014 267 097

## Description

### TECHNICAL FIELD

Embodiments of the present invention relate to a multi-sided display.

### BACKGROUND

With the expansion of application fields of display devices, the display device is no longer limited to the need for flat-panel display. Now the device design for achieving multi-sided display has been raised. The multi-sided display device is generally realized by splicing a plurality of flat-panel displays and achieving display in various directions respectively, e.g., a design of a lamp box. In the multi-sided display, images displayed on various display surfaces are relatively independent and the spliced positions of the display surfaces have edges and corners, so that the continuity of the display images in various directions cannot be achieved.

US 2009/082103 A1 discloses electronic consoles having flexible displays. US 2013/155717 A1 discloses a display apparatus. US 2014/0152646 A1 discloses a cubic display and manufacturing method thereof. From US 2007/0285413 A1 it is known a double-sided display. US 2014/0267097 A1 discloses an image display apparatus and a method of controlling the same. WO 2014/007555 A1 refers to a smart TV having health-management monitoring function and health-management monitoring method using same. EP 1 376 199 A1 discloses a multi-display apparatus having an optical path changing device.

### SUMMARY

Embodiments of the present invention provide a multi-sided display, which is used for achieving continuous multi-sided display.

An embodiment of the present invention provides a multi-sided display, which comprises: a first display panel and a second display panel, wherein two opposite edges of the first display panel are respectively bent towards the reverse side of a display area thereof, to allow the first display panel to form a U-shaped structure; two opposite edges of the second display panel are respectively bent towards the reverse side of a display area thereof, to allow the second display panel to form a U-shaped structure; the two edges of the first display panel bent towards the display area thereof are respectively encapsulated with the two edges of the second display panel bent towards the display area thereof to form a closed structure together with a first encapsulation plate and a second encapsulation plate; and each display surface of the multi-sided display is controlled independently; wherein the first encapsulation plate is configured to seal the top of the closed structure and the second encapsulation plate is configured to seal the bottom of the closed structure ; the multi-sided display further comprises at least one function slot and/or physical function key, wherein the function slot and/or the physical function key are/is disposed at an encapsulated position of the edges of the first display panel and the second display panel; or disposed in a non-display area of the first display panel and/or the second display panel; or disposed on a surface of the first encapsulation plate and/or the second encapsulation plate.

For example, in a possible implementation, in the multi-sided display provided by the embodiment of the present invention, the first display panel and the second display panel, the first display panel and the first encapsulation plate, the first display panel and the second encapsulation plate, the second display panel and the first encapsulation plate, and the second display panel and the second encapsulation plate are assembled together by pressing the mutually matched recesses and corresponding projections, gluing the panels or the plates, or fastening the panels or the plates through screws.

For example, in a possible implementation, in the multi-sided display provided by the embodiment of the present invention, the two edges of the first display panel and the second display panel are respectively encapsulated through a third encapsulation plate.

For example, in a possible implementation, in the multi-sided display provided by the embodiment of the present invention, the third encapsulation plates, the first encapsulation plate and the second encapsulation plate form an integrated structure.

For example, in a possible implementation, the multi-sided display provided by the embodiment of the present invention further comprises a health monitoring unit, wherein the health monitoring unit is disposed at an encapsulated position of the edges of the first display panel and the second display panel; or disposed in a non-display area of the first display panel and/or the second display panel; or disposed on a surface of the first encapsulation plate and/or the second encapsulation plate.

For example, in a possible implementation, in the multi-sided display provided by the embodiment of the present invention, the health monitoring unit includes an infrared detection unit and/or a minimally invasive detection unit; the infrared detection unit is configured to detect a body temperature; and the minimally invasive detection unit is configured to detect blood glucose.

For example, in a possible implementation, in the multi-sided display provided by the embodiment of the present invention, the first display panel and the second display panel are frameless at least at the encapsulated positions of the edges.

For example, in a possible implementation, in the multi-sided display provided by the embodiment of the present invention, the first display panel and the second display panel further include a circuit board; and the circuit board of the first display panel and the second display panel is connected with the display panels through flexible electronic skins.

For example, in a possible implementation, in the multi-sided display provided by the embodiment of the present invention, a transparent prism structure is disposed at least at the encapsulated position of the edges of the first display panel and the second display panel and configured to change direction of emergent light.

For example, in a possible implementation, in the multi-sided display provided by the embodiment of the present invention, the transparent prism structure is at least projected out of the outer surface of the closed structure; and a section of the transparent prism structure, perpendicular to the outer surface of the closed structure, is a semicircle or a positive trapezoid.

For example, in a possible implementation, in the multi-sided display provided by the embodiment of the present invention, the first display panel is a liquid crystal display (LCD) panel, an organic light-emitting diode (OLED) display panel or an electronic paper display panel; and the second display panel is an LCD panel, an OLED display panel or an electronic paper display panel.

For example, in a possible implementation, in the multi-sided display provided by the embodiment of the present invention, the first display panel and the second display panel are touch display panels.

### BRIEF DESCRIPTION OF THE DRAWINGS

A brief description will be given below to the accompanying drawings of the embodiments to provide a more clear understanding of the technical solutions of embodiments of the present invention. It is apparent that the drawings described below only involve some embodiments of the present invention but are not intended to limit the present invention.
FIG. 1 is a structural exploded view I of components in a multi-sided display provided by an embodiment of the present invention;
FIG. 2a is a top view of the multi-sided display provided by an embodiment of the present invention;
FIG. 2b is a schematic structural exploded view of the multi-sided display provided by an embodiment of the present invention, in which the recess and projection pressing way is adopted for assembling the components;
FIG. 2c is a schematic structural exploded view of the multi-sided display provided by an embodiment of the present invention, in which the gluing way is adopted for assembling the components;
FIG. 2d is a schematic structural exploded view of the multi-sided display provided by an embodiment of the present invention, in which the screw is adopted for assembling the components;
FIG. 3a is a structural exploded view II of the components of the multi-sided display provided by an embodiment of the present invention;
FIG. 3b is a structural exploded view III of the components of the multi-sided display provided by an embodiment of the present invention;
FIG. 4 is a schematic diagram I of the multi-sided display provided by an embodiment of the present invention comprising a transparent prism structure;
FIG. 5 is a schematic diagram II of the multi-sided display provided by an embodiment of the present invention comprising the transparent prism structure; and
FIG. 6 is a structural exploded view IV of the components of the multi-sided display provided by an embodiment of the present invention.

### DETAILED DESCRIPTION

Technical solutions according to embodiments of the present invention will be described clearly and completely as below in conjunction with the accompanying drawings of the embodiments of the present invention. It is apparent that the described embodiments are only a part of but not all of exemplary embodiments of the present invention. Based on the described embodiments of the present invention, various other embodiments can be obtained by those of ordinary skill in the art without creative labor and those embodiments shall fall into the protection scope of the present invention.

Detailed description will be given below to the specific implementations of the multi-sided display provided by an embodiment of the present invention with reference to the accompanying drawings.

It should be noted that the shape and the size of the components in the accompanying drawings do not reflect the true scale of the multi-sided display and are only intended to illustrate the content of the present invention.

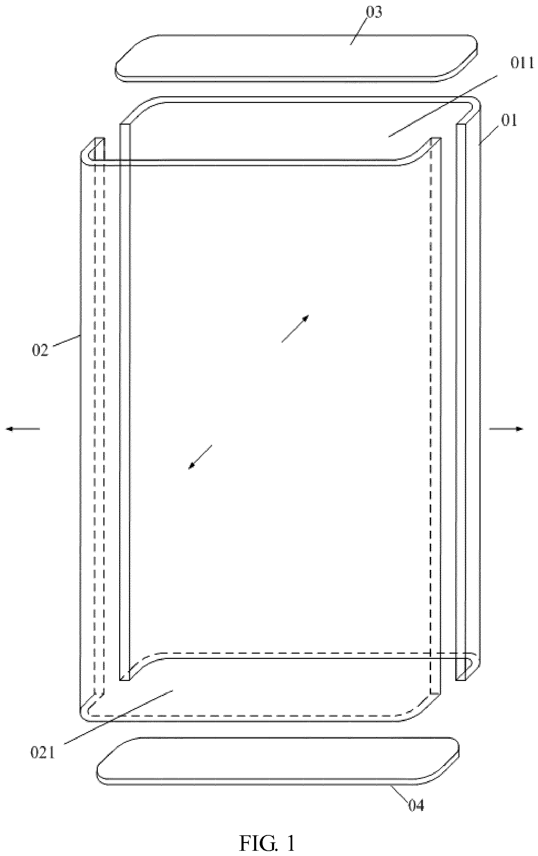

An embodiment of the present invention provides a multi-sided display, which comprises a first display panel 01 and a second display panel 02, as shown in FIG. 1.

As shown in FIG. 1, two opposite edges of the first display panel 01 are respectively bent towards the reverse side of a display area 011 thereof, to allow the first display panel 01 to form a U-shaped structure.

As shown in FIG. 1, two opposite edges of the second display panel 02 are respectively bent towards the reverse side of a display area 021 thereof, to allow the second display panel 02 to form a U-shaped structure.

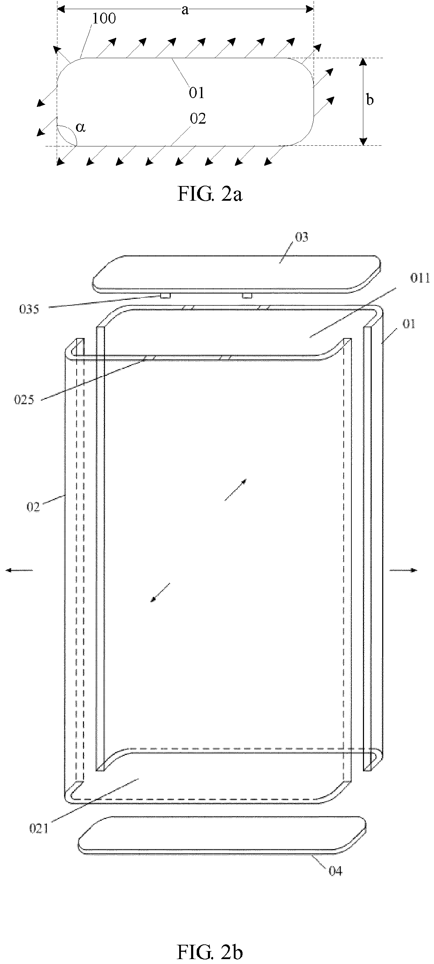

The two edges of the first display panel 01 bent towards the reverse side of the display area thereof are respectively encapsulated with the two edges of the second display panel 02 bent towards the reverse side of the display panel thereof to form a closed structure. As shown by the top view of FIG. 2a, a display surface 100 of the multi-sided display is an outer surface of the closed structure, as shown by the oblique line in FIG. 2a.

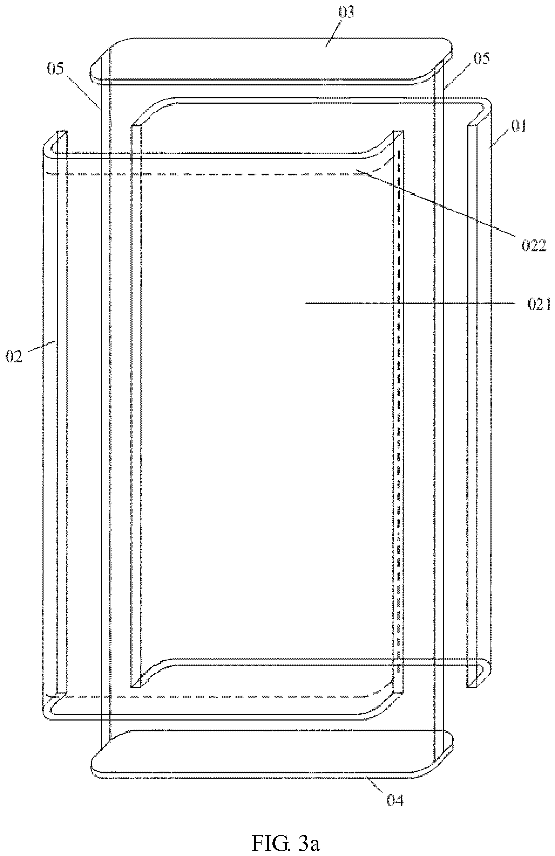

For example, the display surface 100 may include the display area 011 of the first display panel 01 and the display area 021 of the second display panel 02. The display surface may only include the display areas and may also include non-display areas (peripheral areas). The non-display area may be disposed on one side of the display area or may encircle the display area. For example, as shown in FIG. 3a, a non-display area 022 encircles the display area 021 of the second display panel 02.

In a multi-sided display provided by the embodiment of the present invention, the bent first display panel 01 and the bent second display panel 02 are encapsulated into a closed structure. As seen from FIG. 1, the closed structure is composed of four flat surfaces and four cambered or circular arc-like surfaces, and the flat surfaces and the four cambered surfaces in at least four directions (front, back, left and right) of the closed structure can all achieve the function of image display. Moreover, the first display panel 01 and the second display panel 02 adopting the bent U-shaped structures, which can achieve display surfaces in four directions (front, back, left and right), may be combined into a curved surface through the cambered surfaces. Compared with a multi-sided display formed by a plurality of planar display panels, which has a plurality of edges and corners, the multi-sided display herein can enhance the continuity of image display in various directions. It should be noted that, in FIG. 1, an upward sloping arrow indicates a rear surface of the closed structure; a downward sloping arrow indicates a front surface of the closed structure; a left arrow indicates a left surface of the closed structure; and a right arrow indicates a right surface of the closed structure.

In implementation, as shown in FIG. 2a, the proportion of the length b and a of two adjacent flat surfaces in the multi-sided display may be adjusted according to an application scenario of the multi-sided display, so that an external contour of the multi-sided display is like a columnar structure of which a section is a rectangle or a columnar structure of which a section is a square. When the multi-sided display is applied in mobile terminals for displays, such as mobile phones, front and rear surfaces may be set to have large area and left and right surfaces are set to be narrow, so that the ultrathin design of the mobile phones can be conveniently achieved. Moreover, components necessary for mobile terminals, such as power supplies and circuit boards, may be mounted in the closed structure of the multi-sided display, to allow an integrated structure capable of displaying image to be formed on an outer surface of the mobile terminal. When the multi-sided display is applied for three-dimensional (3D) multi-sided display, such as a stereo display wall, the area of front and rear surfaces and left and right surfaces may be set to be substantially same. No further description will be repeated herein.

Moreover, an included angle between two adjacent flat surfaces of the multi-sided display may be adjusted according to actual needs. Generally, for the display surface of the multi-sided display to have good image display continuity, the included angle is generally selected to be an obtuse angle or a right angle. Illustration is given in FIG. 2a by taking the case that the included angle is a right angle as an example.

In implementation, in a multi-sided display provided by an embodiment of the present invention, at least one display surface thereof may be set to be a main display surface according to an application scenario. For example, when the multi-sided display is applied in a mobile phone, the front and rear surfaces may be taken as main display surfaces, and the working state may be as follows.
1. The same content may be simultaneously displayed on the main display surfaces. For example, the same image may be simultaneously displayed on the front and rear surfaces.
2. Different contents may be respectively displayed on a plurality of main display surfaces. For example, an image of the front of the human body is displayed on the front surface and an image of the back of the human body is displayed on the rear surface.
3. The content is only displayed on one main display surface and other main display surfaces are in the off state.

Moreover, in a multi-sided display provided by an embodiment of the present invention, some function keys or user interface (UI) icons of application (APP) software and the like may be displayed on other display surfaces except the main display surfaces according to the settings. For example, function buttons may be displayed on the left and the right surfaces and four curved transition surfaces.

The description on the settings and the working states of the main display surfaces is an illustrated demonstration. In implementation, the same content may be displayed by taking the first display panel 01 and the second display panel 02 as a whole, or different contents may be displayed in different areas. The present invention is not limited thereto.

In implementation, in a multi-sided display provided by an embodiment of the present invention, the first display panel 01 and the second display panel 02 may be formed by touch display panels, so that users can directly adopt the touch display panels for corresponding operations when the function keys are displayed. The first display panel 01 and the second display panel 02 may also be a non-touch display panel. The present invention is not limited thereto.

Moreover, when the multi-sided display is applied in a display wall, the first display panel and the second display panel may also be made as a transparent display panel. In such a way, some articles may be placed in the closed structure formed by the first display panel 01 and the second display panel 02 to be exhibited conveniently, so that the corresponding first display panel 01 and the corresponding second display panel 02 may display relevant information, such as an introduction of the exhibited articles, and relevant browsing is achieved through the touch function of the multi-sided display. When the multi-sided display is applied in a mobile phone, the first display panel and the second display panel are generally set to be a non-transparent display panel.

In implementation, in a multi-sided display provided by an embodiment of the present invention, the first display panel 01 may be an LCD panel, an OLED display panel or an electronic paper display panel. Correspondingly, the second display panel 02 may also be an LCD panel, an OLED display panel or an electronic paper display panel.

Moreover, as the first display panel 01 and the second display panel 02 require bending process, a flexible substrate must be selected when the first display panel 01 and the second display panel 02 are manufactured. The flexible substrate may include a glass substrate, a plastic substrate, or the like. The plastic substrate, for example, may include resin films, or the like, but it is not limited thereto. The materials of corresponding functional layers also must have corresponding flexibility, so that specific bending process of the first display panel 01 and the second display panel 02 can be performed.

Moreover, as shown in FIG. 1, a multi-sided display provided by an embodiment of the present invention may further comprise: a first encapsulation plate 03 for sealing the top of the closed structure and a second encapsulation plate 04 for sealing the bottom of the closed structure. The first encapsulation plate 03 and the second encapsulation plate 04 must have corresponding shapes according to the sectional view of the closed structure, to allow sealing the top and the bottom of the closed structure.

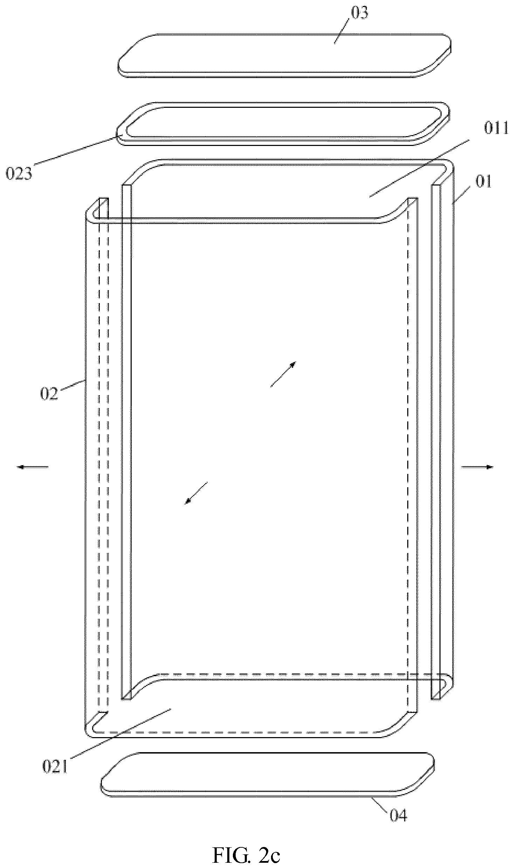

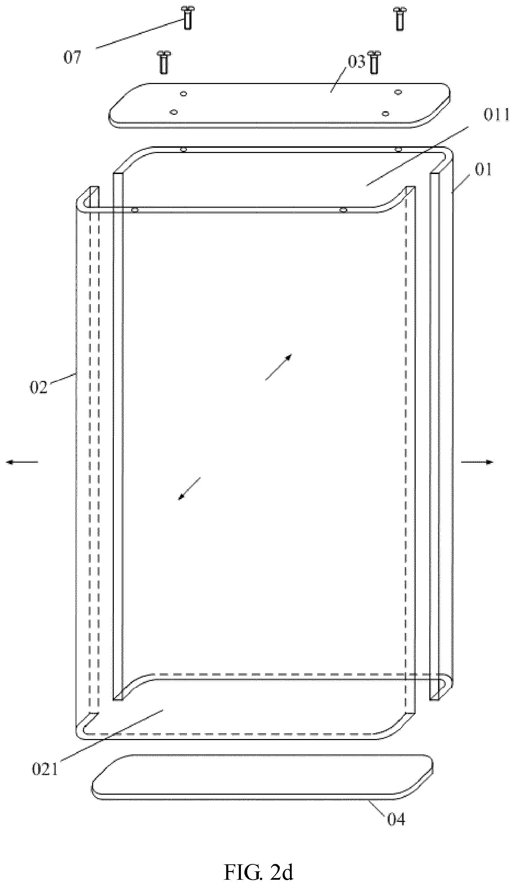

In implementation, when the multi-sided display provided by the embodiments of the present invention is manufactured, the first display panel 01 and the second display panel 02, the first display panel 01 and the first encapsulation plate 03, the first display panel 01 and the second encapsulation plate 04, the second display panel 02 and the first encapsulation plate 03, and the second display panel 02 and the second encapsulation plate 04 may be assembled via a plurality of ways. For example, they may be assembled by pressing the mutually matched recesses and corresponding projections (as shown in FIG. 2b), or gluing together (as shown in FIG. 2c), or fastening by screws 07 (as shown in FIG. 2d). But the present invention is not limited thereto.

For example, when the pressing way of mutually matched recesses and corresponding projections is adopted for the assembling process, recesses may be formed on one component to be assembled and projections may be formed on the other component to be assembled. Taking the assembly of the second display panel 02 and the first encapsulation plate 03 as an example, as shown in FIG. 2b, recesses 025 may be formed on the second display panel 02 and projections 035 are formed on the first encapsulation plate 03, or projections may also be formed on the second display panel 02 and recesses are formed on the first encapsulation plate 03. But the present invention is not limited thereto.

As shown in FIG. 2c, when the gluing way is adopted for the assembling process, an adhesion layer 023 having adhesive effect may be formed between two components to be assembled.

Moreover, in a multi-sided display provided by an embodiment of the present invention, to simplify the complexity of the structure, as shown in FIG. 3a, two edges of the first display panel 01 and the second display panel 02 may also be respectively encapsulated through a third encapsulation plate 05.

Furthermore, the third encapsulation plates 05, the first encapsulation plate 03 and the second encapsulation plate 04 may be set to be an integral structure. In this way, when the multi-sided display provided by the embodiment of the present invention is assembled, the integral structure, the first display panel 01 and the second display panel 02 may be directly assembled.

For example, the adoption of the pressing or gluing way to assemble the first display panel 01 and the second display panel 02 may be favorable for the frameless design of the first display panel 01 and the second display panel 02 at the encapsulated positions of the edges. That is to say, when the first display pane 01 and the second display panel 02 are manufactured, left and right edges thereof may be set to be frameless, to allow the first display panel 01 and the second display panel 02 are at least frameless at the encapsulated positions of the edges, to allow the first display panel 01 and the second display panel 02 to be frameless at the encapsulated positions of the edges.

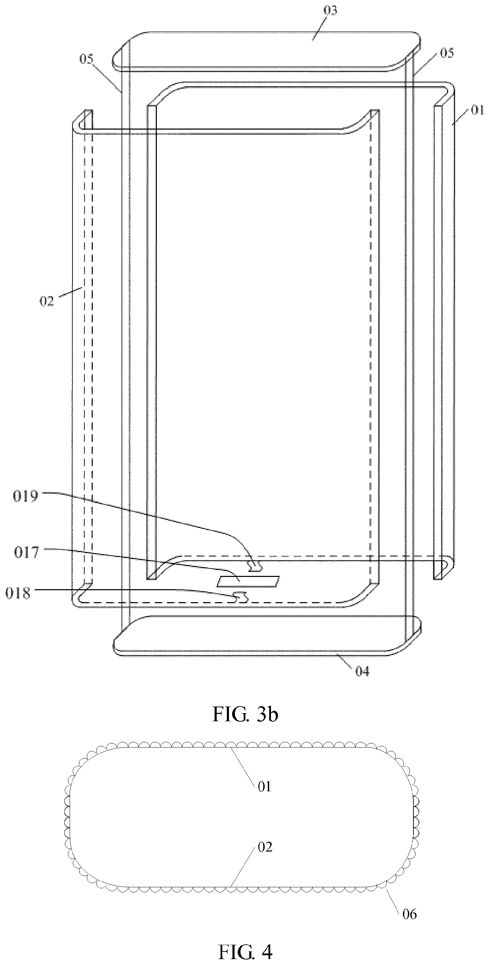

Furthermore, as shown in FIG. 3b, the first display panel and the second display panel generally may further include a circuit board 017. To ensure the connection between the circuit board 017 of the first display panel 01 and the second display panel 02 and the display panels, both (the circuit board and the display panels) may also be connected through flexible electronic skins 018 and 019. The flexible electronic skin generally may be made from meshy conductive rubber materials. When the electronic skin is adopted to connect the display area and the circuit board, upper and lower frames and frames at the encapsulated positions of the edges of the first display panel 01 and the second display panel 02 may all achieve a frameless design, to allow the finally assembled multi-sided display to achieve real frameless circular display.

Moreover, in a multi-sided display provided by an embodiment of the present invention, optical design may also be added, so that viewers cannot view the encapsulated frames of the first display panel 01 and the second display panel 02 visually. For example, as shown in FIG. 4, a transparent prism structure 06 for changing the direction of emergent light may be formed at the encapsulated position of the edges of the first display panel 01 and the second display panel 02. The transparent prism structure 06 may refract light at the encapsulated position of the edges of the first display panel 01 and the second display panel 02, which is originally emitted towards the front, to the encapsulated position of the edges, so that the viewers cannot view seams visually, and the visual effect of frameless circular display can be achieved.

It should be noted that, when the OLED display panel is adopted to manufacture the first display panel and the second display panel, the film encapsulation technology of the OLED display panel may be utilized to achieve physical frameless effect. When the LCD panel or the electronic paper display panel is adopted to manufacture the first display panel and the second display panel, due to the encapsulation technology of the LCD panel and the e-paper display panel, real physical frameless effect cannot be achieved, so some optical designs such as a transparent prism structure must be adopted to help achieving the optical frameless effect. When the OLED display panel is adopted to manufacture the first display panel and the second display panel, some optical designs may also be adopted to help optimizing the frameless effect.

In implementation, a transparent prism structure 06 may be disposed only at the encapsulated position of the edges of the first display panel 01 and the second display panel 02, or as shown in FIG. 4, the transparent prism structure 06 may be disposed on all the outer surfaces of the first display panel 01 and the second display panel 02. But the present invention is not limited thereto.

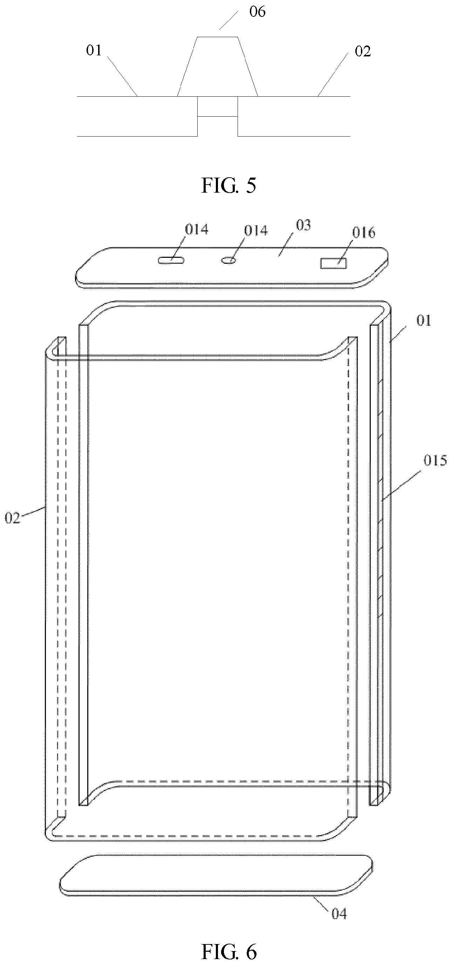

Moreover, the transparent prism structure 06 disposed at the encapsulated position of the edges of the first display panel 01 and the second display panel 02 shall be at least partially projected out of the outer surface of the closed structure, namely shall be at least partially projected out of the outer surfaces of the first display panel 01 and the second display panel 02, as shown in the sectional view FIG. 5. And a section of the transparent prism structure 06, perpendicular to the outer surface of the closed structure, namely the outer surfaces of the first display panel 01 and the second display panel 02, may be set to be a semicircle (as shown in FIG. 4), or a positive trapezoid (as shown in FIG. 5) or the like, so that the light-emitting direction can be changed.

Furthermore, as shown in FIG. 6, a multi-sided display provided by an embodiment of the present invention may further comprise: at least one function slot 015 and/or physical function key 014. For example, the function slot may be an SD card slot, an SIM card slot, a USB slot, a headphone slot, a charging slot, or the like. The physical function key may be a switch button, a sound adjustment button, a brightness adjustment button, or the like. When a multi-sided display provided by an embodiment of the present invention has specific frame, the function slot and/or the physical function key may be disposed at the encapsulated position of the edges of the first display panel 01 and the second display panel 02. For example, the function slot and/or the physical function key may only be disposed at the encapsulated position of one side and may also be disposed at the encapsulated positions of two edges. But the present invention is not limited thereto. The function slot and/or the physical function key may also be disposed in the non-display area(s) of the first display panel 01 and/or the second display panel 02. When a multi-sided display provided by an embodiment of the present invention is frameless, the function slot and/or the physical function key may be disposed on the surface(s) of the first encapsulation plate 03 and/or the second encapsulation plate 04. FIG. 6 illustrates two physical function keys 014 disposed on the first encapsulation plate 03 and a plurality of function slots 015 formed at the encapsulated position of one side of the first display panel 01 and the second display panel 02.

Furthermore, a multi-sided display provided by an embodiment of the present invention has the function of multi-sided display and may also be provided with some additional features to improve the product merit. For example, in implementation, a multi-sided display provided by an embodiment of the present invention may further comprise: a health monitoring unit 016. The health monitoring unit may be configured to detect the health of the human body and may also realize telemedicine and achieve corresponding display on the display surface of the multi-sided display.

When the multi-sided display provided by the embodiment of the present invention has specific frame, the health monitoring unit is disposed at the encapsulated position of the edges of the first display panel 01 and the second display panel 02. For example, the health monitoring unit may only be disposed at the encapsulated position of one side and may also be disposed at the encapsulated positions of two edges. But the present invention is not limited thereto. The health monitoring unit may also be disposed in the non-display area(s) of the first display panel 01 and/or the second display panel 02. When a multi-sided display provided by an embodiment of the present invention is frameless, the health monitoring unit may be disposed on the surface(s) of the first encapsulation plate 03 and/or the second encapsulation plate 04.

Generally, the health monitoring unit is provided with a data acquisition component and a data analysis and storage component. For example, the data acquisition unit generally may adopt infrared sampling, sensor sampling, or the like. For example, the health monitoring unit may adopt an infrared detection unit and/or a minimally invasive detection unit. For example, the infrared detection unit may be configured to monitor the body temperature, and the minimally invasive detection unit may be configured to monitor the blood glucose.

Furthermore, when a multi-sided display provided by an embodiment of the present invention is applied in products, such as IT display products, TVs and mobile phones, image acquisition devices, such as cameras, and devices, such as bluetooth devices, may also be disposed in the non-display area(s) of the first display panel 01 and/or the second display panel 02. But the present invention is not limited thereto.

The multi-sided display provided by the embodiments of the present invention comprises a first display panel and a second display panel. Two opposite edges of the first display panel and the second display panel are respectively bent towards the reverse sides of display areas, to allow the first display panel and the second display panel to form U-shaped structures. The two edges of the first display panel bent towards the reverse side of the display area thereof are respectively encapsulated with the two edges of the second display panel bent towards the reverse side of the display area thereof to form a closed structure. A display surface of the multi-sided display is an outer surface of the closed structure. In the multi-sided display provided by the embodiments of the present invention, the bent first display panel and the bent second display panel are encapsulated to form a closed structure, and the function of image display can be achieved on surfaces in at least four directions of the closed structure. And in the first display panel and the second display panel adopting the bent U-shaped structures, display surfaces in four directions may be combined into a curved surface through cambered or circular arc-like surfaces. Compared with a multi-sided display formed by a plurality of planar display panels, which has a plurality of edges and corners, the multi-sided display can enhance the continuity of image display in various directions.

The following points should be noted:
(1) Unless otherwise specified, the technical terms or scientific terms used herein have normal meanings understood by those skilled in the art. The words "first", "second" and the like used in the disclosure do not indicate the sequence, the number or the importance but are only used for distinguishing different components.
(2) The accompanying drawings in the embodiments of the present invention only involve relevant structures of the embodiments of the present invention, and other structures may refer to general designs.
(3) The embodiments of the present invention and the characteristics in the embodiments may be combined with each other on the premise of no conflict.

The described above are just exemplary implementations of the present invention and the invention is not intended to be limited thereto. An ordinary skill in the art can contemplate various variations and modifications to the present invention without departure from the scope of the present invention, and such variations and modifications shall fall in the scope of the present invention. The scope of the present invention is defined by the claims.

## Claims

1. A multi-sided display, comprising: a first display panel (01) and a second display panel (02),
two opposite edges of the first display panel (01) are respectively bent towards the reverse side of a display area (011) thereof, to allow the first display panel (01) to form a U-shaped structure;
two opposite edges of the second display panel (02) are respectively bent towards the reverse side of a display area (021) thereof, to allow the second display panel (02) to form a U-shaped structure;
the two edges of the first display panel (01) bent towards the display area (011) thereof are respectively encapsulated with the two edges of the second display panel (02) bent towards the display area (021) thereof to form a closed structure together with a first encapsulation plate (03) and a second encapsulation plate (04); and each display surface of the multi-sided display is controlled independently ;
; wherein the first encapsulation plate (03) is configured to seal the top of the closed structure and the second encapsulation plate (04) is configured to seal the bottom of the closed structure ;
the multi-sided display further comprises at least one function slot (015) and/or physical function key (014), wherein the function slot (015) and/or the physical function key (014) are/is disposed at an encapsulated position of the edges of the first display panel (01) and the second display panel (02); or disposed in a non-display area of the first display panel (01) and/or the second display panel (02); or disposed on a surface of the first encapsulation plate (03) and/or the second encapsulation plate (04).

2. The multi-sided display according to claim 1, wherein the first display panel (01) and the second display panel (02), the first display panel (01) and the first encapsulation plate (03), the first display panel (01) and the second encapsulation plate (04), the second display panel (02) and the first encapsulation plate (03), and the second display panel (02) and the second encapsulation plate (04) are assembled together by pressing the mutually matched recesses and corresponding projections, gluing the panels or the plates, or fastening the panels or the plates through screws.

3. The multi-sided display according to claim 1, wherein the two edges of the first display panel (01) and the two edges of the second display panel (02) are respectively encapsulated through a third encapsulation plate (05).

4. The multi-sided display according to claim 3, wherein the third encapsulation plates (05), the first encapsulation plate (03) and the second encapsulation plate (04) are an integrated structure.

5. The multi-sided display according to claim 1, further comprising a health monitoring unit (016), wherein
the health monitoring unit (016) is disposed at an encapsulated position of the edges of the first display panel (01) and the second display panel (02); or disposed in a non-display area of the first display panel (01) and/or the second display panel (02); or disposed on a surface of the first encapsulation plate (03) and/or the second encapsulation plate (04).

6. The multi-sided display according to claim 5, wherein the health monitoring unit (016) includes an infrared detection unit and/or a minimally invasive detection unit;
the infrared detection unit is configured to detect a body temperature; and
the minimally invasive detection unit is configured to detect blood glucose.

7. The multi-sided display according to any one of claims 1 to 6, wherein the first display panel (01) and the second display panel (02) are at least frameless at the encapsulated positions of the edges.

8. The multi-sided display according to claim 7, wherein the first display panel (01) and the second display panel (02) further include a circuit board; and the circuit board of the first display panel (01) and the second display panel (02) is connected with the display panels through flexible electronic skins.

9. The multi-sided display according to claim 7, wherein a transparent prism structure (06) is at least disposed at the encapsulated position of the edges of the first display panel (01) and the second display panel (02) and configured to change the direction of emergent light.

10. The multi-sided display according to claim 9, wherein the transparent prism structure (06) is at least projected out of the outer surface of the closed structure; and
a section of the transparent prism structure (06), perpendicular to the outer surface of the closed structure, is a semicircle or a positive trapezoid.

11. The multi-sided display according to any one of claims 1-10, wherein the first display panel (01) is a liquid crystal display (LCD) panel, an organic light-emitting diode (OLED) display panel or an electronic paper display panel; and
the second display panel (02) is an LCD panel, an OLED display panel or an electronic paper display panel.

12. The multi-sided display according to claim 11, wherein the first display panel (01) and the second display panel (02) are touch display panels.

## Patentansprüche

1. Mehrseitiges Display, umfassend: ein erstes Display-Feld (01) und ein zweites Display-Feld (02),
wobei zwei sich gegenüberliegende Ränder des ersten Display-Feldes (01) jeweils in Richtung zu der Rückseite von einem Display-Bereich (011) davon gebogen sind, um dem ersten Display-Feld (01) zu ermöglichen, eine U-förmige Struktur zu bilden;
wobei zwei sich gegenüberliegende Ränder des zweiten Display-Feldes (02) jeweils in Richtung zu der Rückseite eines Display-Bereiches (021) davon gebogen sind, um dem zweiten Display-Feld (02) zu ermöglichen, eine U-förmige Struktur zu bilden;
wobei die zwei Ränder von dem ersten Display-Feld (01), die zu dem Display-Bereich (011) davon gebogen sind, jeweils eingekapselt sind mit den zwei Rändern des zweiten Display-Feldes (02), die zu dem Display-Bereich (021) davon gebogen sind, um mit einer ersten Einkapselungsplatte (03) und einer zweiten Einkapselungsplatte (04) eine geschlossene Struktur zu bilden; und wobei jede Display-Oberfläche des mehrseitigen Displays unabhängig gesteuert ist;
wobei die erste Einkapselungsplatte (03) konfiguriert ist, um das obere Ende der geschlossenen Struktur zu verschließen, und wobei die zweite Einkapselungsplatte (04) konfiguriert ist, um das untere Ende der geschlossenen Struktur zu verschließen;
wobei das mehrseitige Display außerdem zumindest einen Funktions-Slot (015) und/oder physikalischen Funktionsschlüssel (014) umfasst, wobei der Funktions-Slot (015) und/oder der physikalische Funktionsschlüssel (014) angeordnet ist/sind bei einer eingekapselten Position der Ränder des ersten Display-Feldes (01) und des zweiten Display-Feldes (02); oder angeordnet in einem Nicht-Display-Bereich des ersten Display-Feldes (01) und/oder des zweiten Display-Feldes (02); oder angeordnet auf einer Oberfläche der ersten Einkapselungsplatte (03) und/oder der zweiten Einkapselungsplatte (04).

2. Mehrseitiges Display gemäß Anspruch 1, wobei das erste Display-Feld (01) und das zweite Display-Feld (02), das erste Display-Feld (01) und die erste Einkapselungsplatte (03), das erste Display-Feld (01) und die zweite Einkapselungsplatte (04), das zweite Display-Feld (02) und die erste Einkapselungsplatte (03), und das zweite Display-Feld (02) und die zweite Einkapselungsplatte (04) zusammengebaut sind mittels Pressen der gegenseitig passenden Aussparungen und korrespondierenden Vorsprünge, Kleben der Felder oder der Platten oder Befestigen der Felder oder der Platten durch Schrauben.

3. Mehrseitiges Display gemäß Anspruch 1, wobei die zwei Ränder des ersten Display-Feldes (01) und die zwei Ränder des zweiten Display-Feldes (02) jeweils durch eine dritte Einkapselungsplatte (05) eingekapselt sind.

4. Mehrseitiges Display gemäß Anspruch 3, wobei die dritte Einkapselungsplatte (05), die erste Einkapselungsplatte (03) und die zweite Einkapselungsplatte (04) eine integrierte Struktur sind.

5. Mehrseitiges Display gemäß Anspruch 1, außerdem umfassend eine Gesundheitsüberwachungseinheit (016), wobei die Gesundheitsüberwachungseinheit (016) bei einer eingekapselten Position der Ränder des ersten Display-Feldes (01) und des zweiten Display-Feldes (02) angeordnet ist; oder angeordnet ist in einem Nicht-Display-Bereich des ersten Display-Feldes (01) und/oder des zweiten Display-Feldes (02);
oder angeordnet ist auf einer Oberfläche der ersten Einkapselungsplatte (03) und/oder der zweiten Einkapselungsplatte (04).

6. Mehrseitiges Display gemäß Anspruch 5, wobei die Gesundheitsüberwachungseinrichtung (016) eine Infrarotdetektionseinheit und/oder eine minimalinvasive Detektionseinheit enthält;
wobei die Infrarotdetektionseinheit konfiguriert ist, eine Körpertemperatur zu detektieren; und
wobei die minimalinvasive Detektionseinheit konfiguriert ist, Blutzucker zu detektieren.

7. Mehrseitiges Display gemäß einem der Ansprüche 1 bis 6, wobei das erste Display-Feld (01) und das zweite Display-Feld (02) zumindest rahmenlos bei den eingekapselten Positionen der Ränder sind.

8. Mehrseitiges Display gemäß Anspruch 7, wobei das erste Display-Feld (01) und das zweite Display-Feld (02) außerdem zumindest eine Schaltungsplatine enthalten; und wobei die Schaltungsplatine des ersten Display-Feldes (01) und des zweiten Display-Feldes (02) mit den Display-Feldern durch flexible elektronische Häute verbunden ist.

9. Mehrseitiges Display gemäß Anspruch 7, wobei eine transparente Prismenstruktur (06) zumindest an der eingekapselten Position der Ränder des ersten Display-Feldes (01) und des zweiten Display-Feldes (02) angeordnet ist und konfiguriert ist, die Richtung von austretendem Licht zu verändern.

10. Mehrseitiges Display gemäß Anspruch 9, wobei die transparente Prismenstruktur (06) zumindest nach außen über die äußere Oberfläche der geschlossenen Struktur hervorsteht; und wobei ein Abschnitt der transparenten Prismenstruktur (06), senkrecht zu der äußeren Oberfläche der geschlossenen Struktur, ein Halbkreis oder ein positives Trapez ist.

11. Mehrseitiges Display gemäß einem der Ansprüche 1-10, wobei das erste Display-Feld (01) ein Flüssigkristalldisplay (LCD)-Feld, ein organisches-Licht-emittierendes Dioden (OLED)-Display-Feld oder ein elektronisches Papier-Display-Feld ist; und
wobei das zweite Display-Feld (02) ein LCD-Feld, ein OLED-Display-Feld oder ein elektronisches Papier-Display-Feld ist.

12. Mehrseitiges Display gemäß Anspruch 11, wobei das erste Display-Feld (01) und das zweite Display-Feld (02) Berührungs-Display-Felder sind.

## Revendications

1. Dispositif d'affichage à côtés multiples, comprenant :
un premier panneau d'affichage (01) et un second panneau d'affichage (02),
deux bords opposés du premier panneau d'affichage (01) sont respectivement courbés en direction du côté inverse d'une zone d'affichage (011) de celui-ci, pour permettre au premier panneau d'affichage (01) de former une structure en U ;
deux bords opposés du second panneau d'affichage (02) sont respectivement courbés en direction du côté inverse d'une zone d'affichage (021) de celui-ci, pour permettre au second panneau d'affichage (02) de former une structure en U ;
les deux bords du premier panneau d'affichage (01) courbés en direction de la zone d'affichage (011) de celui-ci sont respectivement encapsulés avec les deux bords du second panneau d'affichage (02) courbés en direction de la zone d'affichage (021) de celui-ci pour former une structure fermée conjointement avec une première plaque d'encapsulage (03) et une deuxième plaque d'encapsulage (04) ; et chaque surface d'affichage de l'affichage à côtés multiples est commandée indépendamment ;
dans lequel la première plaque d'encapsulage (03) est configurée pour fermer hermétiquement le dessus de la structure fermée et la deuxième plaque d'encapsulage (04) est configurée pour fermer hermétiquement le fond de la structure fermée ;
l'affichage à côtés multiples comprend en outre au moins une fente fonctionnelle (015) et/ou une clé fonctionnelle physique (014), dans lequel la fente fonctionnelle (015) et/ou la clé fonctionnelle physique (014) est/sont disposée(s) à une position encapsulée des bords sur le premier panneau d'affichage (01) et le second panneau d'affichage (02) ; ou disposée (s) dans une zone de non-affichage du premier panneau d'affichage (01) et/ou du second panneau d'affichage (02) ; ou disposée(s) sur une surface de la première plaque d'encapsulage (03) et/ou de la deuxième plaque d'encapsulage (04).

2. Dispositif d'affichage à côtés multiples selon la revendication 1, dans lequel le premier panneau d'affichage (01) et le second panneau d'affichage (02), le premier panneau d'affichage (01) et la première plaque d'encapsulage (03), le premier panneau d'affichage (01) et la deuxième plaque d'encapsulage (04), le second panneau d'affichage (02) et la première plaque d'encapsulage (03), et le second panneau d'affichage (02) et la deuxième plaque d'encapsulage (04) sont assemblés ensemble en pressant les creux et saillies correspondantes appariés mutuellement, en collant les panneaux ou les plaques ou en fixant les panneaux ou les plaques par des vis.

3. Dispositif d'affichage à côtés multiples selon la revendication 1, dans lequel les deux bords du premier panneau d'affichage (01) et les deux bords du second panneau d'affichage (02) sont respectivement encapsulés par une troisième plaque d'encapsulage (05) .

4. Dispositif d'affichage à côtés multiples selon la revendication 3, dans lequel la troisième plaque d'encapsulage (05), la première plaque d'encapsulage (03) et la seconde plaque d'encapsulage (04) sont une structure intégrée.

5. Dispositif d'affichage à côtés multiples selon la revendication 1, comprenant en outre une unité de surveillance de santé (016), dans lequel
l'unité de surveillance de santé (016) est disposée dans une position encapsulée des bords du premier panneau d'affichage (01) et du second panneau d'affichage (02) ; ou disposé dans une zone de non-affichage du premier panneau d'affichage (01) et du second panneau d'affichage (02) ; ou disposé sur une surface de la première plaque d'encapsulage (03) et de la deuxième plaque d'encapsulage (04).

6. Dispositif d'affichage à côtés multiples selon la revendication 5, dans lequel l'unité de surveillance de santé (016) inclut une unité de détection d'infrarouges et/ou une unité de détection à invasion minimale ;
l'unité de détection d'infrarouges est configurée pour détecter une température du corps ; et
l' unité de détection à invasion minimale est configurée pour détecter la glycémie.

7. Dispositif d'affichage à côtés multiples selon l'une quelconque des revendications 1 à 6, dans lequel le premier panneau d'affichage (01) et le second panneau d'affichage (02) sont au moins sans cadre aux positions encapsulées des bords.

8. Dispositif d'affichage à côtés multiples selon la revendication 7, dans lequel le premier panneau d'affichage (01) et le second panneau d'affichage (02) incluent en outre une carte de circuit ; et la carte de circuit du premier panneau d'affichage (01) et du second panneau d'affichage (02) est connectée aux panneaux d'affichage par des pellicules électroniques flexibles.

9. Dispositif d'affichage à côtés multiples selon la revendication 7, dans lequel une structure à prismes transparents (06) est au moins disposée dans la position encapsulée des bords du premier panneau d'affichage (01) et du second panneau d'affichage (02) et configurée pour changer la direction de la lumière émergente.

10. Dispositif d'affichage à côtés multiples selon la revendication 9, dans lequel la structure à prismes transparents (06) est au moins projetée hors de la surface extérieure de la structure fermée ; et une section de la structure à prismes transparents (06), perpendiculaire à la surface extérieure de la structure fermée, est un demi-cercle ou un trapézoïde positif.

11. Dispositif d'affichage à côtés multiples selon l'une quelconque des revendications 1 - 10, dans lequel le premier panneau d'affichage (01) est un panneau d'affichage à cristaux liquides (LCD), un panneau d'affichage à diode électroluminescente organique (OLED) ou un panneau d'affichage papier électronique ; et
le second panneau d'affichage (02) est un panneau LCD, un panneau d'affichage OLED ou un panneau d'affichage papier électronique.

12. Dispositif d'affichage à côtés multiples selon la revendication 11, dans lequel le premier panneau d'affichage (01) et le second panneau d'affichage (02) sont des panneaux d'affichage tactiles.
